# EUROPEAN PATENT APPLICATION

(11) **EP 4 491 730 A1**
(43) Date of publication of application: **15.01.2025**
(21) Application number: 23306176.1
(22) Date of filing: 10.07.2023
(51) Int. Cl.: C12N 15/52, C12N 9/04, C12N 9/02, C12N 9/16, C12P 7/06, C12P 7/18

(54) **MICROORGANISMS WITH IMPROVED GLYCEROL AND ACETATE PRODUCTION AND THE USE THEREOF FOR 1,3-PROPANEDIOL PRODUCTION**

(71) Applicant: Metabolic Explorer, 63360 Saint Beauzire (FR)
(72) Inventor: JACOTTIN, PAUL, 31400 TOULOUSE (FR); WILDING-STEELE, Tom, 31077 TOULOUSE CEDEX 4 (FR); SOUCAILLE, Philippe, 31450 DEYME (FR)
(74) Representative: Regimbeau

(57) **Abstract**

The present invention concerns a method to produce glycerol and acetate, by culturing at least a new mutant strain of *Clostridium* in an appropriate culture medium.

The invention also relates to said new mutant strain of *Clostridium* which comprises an overexpression of at least one of glycerol phosphate phosphatase and glycerol phosphate dehydrogenase activity. This modified strain may also be adapted and be used for production of 1,3-propanediol from glucose or the same.

## Description

The present invention concerns a method to produce glycerol and acetate, by culturing at least a new mutant strain of *Clostridium* in an appropriate culture medium.

The invention also relates to said new mutant strain of *Clostridium* which comprise an overexpression of at least one of glycerol phosphate phosphatase and glycerol phosphate dehydrogenase activity. This modified strain may also be adapted and be used for production of 1,3-propanediol from glucose or the same.

### BACKGROUND OF THE INVENTION

Glycerol is used in many industries such as cosmetics, paint, automotive, food, and pharmaceutical industries. Currently, glycerol is available in commercial quantities as a by-product from biodiesel production, but the purity and the cost of its purification are prohibitive.

Also, as the mondial glycerol stock is limited, there is a need to find alternative solution or means for producing glycerol.

Fermentative production of glycerol by culturing microorganisms is known in the art. However, such a production by aerobic processes is a high-cost production.

Due to its ability to grow under anaerobic conditions, *Clostridium* genus microorganisms represent an ideal system for the development of such a new biotechnological platform.

Furthermore, increasing glycerol production could be particularly attractive for production of 1,3-propanediol (PDO) which is mainly produced from glycerol. PDO may indeed also be used for many synthesis reactions as a bifunctional organic compound, in particular as a monomer for polycondensations to produce polyesters, polyethers, polyurethanes, and in particular, polytrimethylene terephtalate (PTT). These structure and reactivity features lead to several applications in cosmetics, textiles (clothing fibers or flooring) or plastics (car industry and packing or coating).

In the present patent application, a new biological pathway in *Clostridium* is disclosed leading to increase glycerol production and accumulation from glucose. Thus, the present invention provides a mutant *Clostridium* strain comprising an overexpression of at least one of glycerol phosphate phosphatase and glycerol phosphate dehydrogenase activity, preferably both.

Furthermore, culturing this new mutant strain of *Clostridium* in an appropriate culture medium conducts to an improved production of glycerol, acetate and PDO from glucose.

### BRIEF DESCRIPTION OF THE INVENTION

The present invention concerns a method for the production of glycerol and acetate comprising culturing, in an appropriate culture medium, a *Clostridium* mutant strain comprising an overexpression of at least one of glycerol phosphate phosphatase and glycerol phosphate dehydrogenase activity, preferably both.

Particularly, the method according to the present invention comprises culturing a mutant strain wherein the glycerol phosphate phosphatase is encoded by the gene *CA_C1625* at the locus 1764289 in the *C*. *acetobutylicum* genome ATCC824, Genbank AE001437 with SEQ ID NO: 1. Similarly, the method according to the present invention comprises culturing a mutant strain wherein the glycerol phosphate dehydrogenase is preferably encoded by the gene *CA_C1626* at the locus 1764945 in the *C*. *acetobutylicum* genome ATCC824, Genbank AE001437 with SEQ ID NO: 3.

In a particular embodiment, the method of the invention comprises culturing a *Clostridium* mutant strain wherein at least one gene encoding an enzyme involved in production of metabolites from glycerol is deleted. Preferably, the method of the invention comprises culturing a *Clostridium* mutant strain wherein at least one of the following genes is deleted:
- *ptb* gene encoding phospho-transbutyrylase to avoid butyrate formation,
- *buk* gene encoding butyrate kinase to avoid butyrate formation,
- *ldh* gene encoding lactate dehydrogenase to avoid lactate formation,
- *adhE1* and *adhE2* genes encoding aldehyde-alcohol dehydrogenase to avoid ethanol and butanol formation.

Preferably, the method of the invention comprises culturing a *Clostridium* mutant strain wherein the *adhE1* and *adhE2* genes are deleted by curing the *Clostridium* mutant strain for the pSOL1 megaplasmid.

In a particular embodiment, the method of the invention comprises culturing a *Clostridium* mutant strain which is modified to convert acetate to acetone by overexpressing at least one gene selected among:
- *ctfA* gene from a solventogenic Clostridium and encoding CoA-transferase subunit A, preferably from *Clostridium beijerinckii,*
- *ctfB* gene from a solventogenic *Clostridium* and encoding CoA-transferase subunit B, preferably from *Clostridium beijerinckii,*
- adc gene from a solventogenic *Clostridium* and encoding coenzyme A-acylating aldehyde dehydrogenase, preferably from *Clostridium beijerinckii.*

Preferably, the *hbd* gene encoding 3-hydroxybutyryl-CoA dehydrogenase is also deleted in the *Clostridium* mutant strain.

In a particular embodiment, the method of the invention comprises culturing a *Clostridium* mutant strain which is modified to produce 1,3-propanediol from glycerol by overexpressing the genes:
- *dhaB1* gene from *Clostridium butyricum* and encoding B12-independent glycerol dehydratase,
- *dhaB2* gene from *Clostridium butyricum* and encoding glycerol dehydratase activator, and
- either *dhaT* gene from *Clostridium butyricum* and encoding 1,3-propanediol dehydrogenase or both *bdhb* gene from *Clostridium acetobutylicum* encoding an NADP+ dependent alcohol dehydrogenase and *CA_C0764* gene from *Clostridium acetobutylicum* encoding a Fd-NADP+ reductase.

Preferably, the *hydA* gene encoding hydrogenase I is attenuated in the *Clostridium* mutant strain, more preferably deleted.

In a particular embodiment, the method of the invention comprises culturing a *Clostridium* mutant strain selected from the group consisting of *Clostridium acetobutylicum, Clostridium butyricum, Clostridium beijerinckii, Clostridium pasteurianum and Clostridium thermocellum.*

Preferably, the source of carbon in the culture medium is glucose, fructose, xylose and/or sucrose, more preferably glucose.

The present invention also concerns a mutant strain of *Clostridium* comprising an overexpression of at least one of glycerol phosphate phosphatase encoded by the gene *CA_C1625* at the locus 1764289 in the *C*. *acetobutylicum* genome ATCC824, Genbank AE001437 with SEQ ID NO: 1 and glycerol phosphate dehydrogenase encoded by the gene *CA_C1626* at the locus 1764945 in the *C*. *acetobutylicum* genome ATCC824, Genbank AE001437 with SEQ ID NO: 3, preferably both.

In a particular embodiment of the invention, the *Clostridium* mutant strain further comprise a deletion of at least one gene encoding an enzyme involved in production of metabolites from glycerol. Preferably, a deletion of at least one gene chosen among:
- *ptb* gene encoding phospho-transbutyrylase to avoid butyrate formation,
- *buk* gene encoding butyrate kinase to avoid butyrate formation,
- *ldh* gene encoding lactate dehydrogenase to avoid lactate formation,
- *adhE1* and *adhE2* genes encoding aldehyde-alcohol dehydrogenase to avoid ethanol and butanol formation.

Preferably, the *adhE1* and *adhE2* genes are deleted by curing the *Clostridium* mutant strain for the pSOL1 megaplasmid.

In a particular embodiment, the *Clostridium* mutant strain of the invention is modified to convert acetate to acetone by overexpressing at least one gene selected among:
- *ctfA* gene from a solventogenic Clostridium and encoding CoA-transferase subunit A, preferably from *Clostridium beijerinckii,*
- *ctfB* gene from a solventogenic *Clostridium* and encoding CoA-transferase subunit B, preferably from *Clostridium beijerinckii,* and
- adc gene from a solventogenic *Clostridium* and encoding CoA-acylating aldehyde dehydrogenase, preferably from *Clostridium beijerinckii.*

Preferably, the *hbd* gene encoding 3-hydroxybutyryl-CoA dehydrogenase is also deleted in the *Clostridium* mutant strain.

In a particular embodiment, the *Clostridium* mutant strain of the invention is modified to produce 1,3-propanediol from glycerol by overexpressing the genes:
- *dhaB1* gene from *Clostridium butyricum* and encoding B12-independent glycerol dehydratase,
- *dhaB2* gene from *Clostridium butyricum* and encoding glycerol dehydratase activator, and
- either *dhaT* gene from *Clostridium butyricum* and encoding 1,3-propanediol dehydrogenase or both *bdhb* gene from *Clostridium acetobutylicum* encoding an NADP+ dependent alcohol dehydrogenase and *CA_C0764* gene from *Clostridium acetobutylicum* encoding a Fd-NADP+ reductase.

Preferably, the *hydA* gene encoding hydrogenase I is attenuated in the *Clostridium* mutant strain, more preferably deleted.

In a particular embodiment, the *Clostridium* mutant strain of the invention is selected from the group consisting of *Clostridium acetobutylicum, Clostridium butyricum, Clostridium beijerinckii, Clostridium pasteurianum and Clostridium thermocellum.*

### DETAILLED DESCRIPTION OF THE INVENTION

Before describing the present invention in detail, it is to be understood that this invention is not limited to particularly exemplified methods and may, of course, vary. It is also to be understood that the terminology used herein is for the purpose of describing particular embodiments of the invention only, and is not intended to be limiting, which will be limited only by the appended claims.

All publications, patents and patent applications cited herein, whether supra or infra, are hereby incorporated by reference in their entirety. However, publications mentioned herein are cited for the purpose of describing and disclosing the protocols, reagents and vectors that are reported in the publications and that might be used in connection with the invention. Nothing herein is to be construed as an admission that the invention is not entitled to antedate such disclosure by virtue of prior invention.

Furthermore, the practice of the present invention employs, unless otherwise indicated, conventional microbiological and molecular biological techniques within the skill of the art. Such techniques are well known to the skilled worker, and are explained fully in the literature.

It must be noted that as used herein and in the appended claims, the singular forms "a," "an," and "the," include plural reference unless the context clearly dictates otherwise. Thus, for example, a reference to "a microorganism" includes a plurality of such microorganisms, and a reference to "an endogenous gene" is a reference to one or more endogenous genes, and so forth. Unless defined otherwise, all technical and scientific terms used herein have the same meanings as commonly understood by one of ordinary skill in the art to which this invention belongs. Although any material and methods similar or equivalent to those described herein can be used to practice or test the present invention, the preferred material and methods are now described. As used herein, the following terms may be used for interpretation of the claims and specification.

In the claims which follow and in the preceding description of the invention, except where the context requires otherwise due to express language or necessary implication, the word "comprise" or variations such as "comprises" or "comprising" is used in an inclusive sense, i.e. to specify the presence of the stated features but not to preclude the presence or addition of further features in various embodiments of the invention.

The terms "mutant strain", "mutant microorganism", "recombinant strain" or "recombinant microorganism" are used interchangeably herein and refer to a microorganism or a strain of microorganism that has been genetically modified or genetically engineered. This means, according to the usual meaning of these terms, that the microorganism of the invention is not found in nature and is genetically modified when compared to the "parental" microorganism from which it is derived. The "parental" microorganism may occur in nature (i.e. a wild type microorganism) or may have been previously modified, but does not express or over-express the one or more proteins of the present invention (i.e. glycerol phosphate phosphatase and/or glycerol phosphate dehydrogenase). Accordingly, the *Clostridium* mutant strain of the invention is defined as a *Clostridium* strain which have been modified to express or over-express at least the glycerol phosphate phosphatase and/or glycerol phosphate dehydrogenase proteins that were not expressed or over-expressed in the parental unmodified strain.

Preferably, the parental microorganism is selected from the microorganisms listed herein. In a particular embodiment, the parental microorganism is selected from the *Clostridium* species *C. acetobutylicum, C. butyricum, Clostridium beijerinckii, Clostridium pasteurianum,* and *Clostridium thermocellum* and related isolates. More preferably, the parental microorganism is a *C*. *acetobutylicum* strain. Also, it is well known by the person skilled in the art what are solventogenic *Clostridium* strains, among which *Clostridium beijerinckii* may for example be cited.

The expression "genetically modified" means that the strain has been transformed in the aim to change its genetic characteristics. Endogenous genes can be attenuated, deleted, or over-expressed, or preferably exogenous genes can be introduced, carried by a plasmid, or integrated into the genome of the strain, to be expressed into the cell.

The term "plasmid" or "vector" as used herein refers to an extra chromosomal element often carrying genes which are not part of the central metabolism of the cell, and usually in the form of circular double-stranded DNA molecules.

A microorganism may notably be modified to modulate the expression level of an endogenous gene or the level of production of the corresponding protein or the activity of the corresponding enzyme. The term "endogenous gene" means that the gene was present in the microorganism before any genetic modification. Endogenous genes may be overexpressed by introducing heterologous sequences in addition to, or to replace, endogenous regulatory elements. Endogenous genes may also be overexpressed by introducing one or more supplementary copies of the gene into the chromosome or on a plasmid. In this case, the endogenous gene initially present in the microorganism may be deleted. Endogenous gene expression levels, protein production levels, or the activity of the encoded protein, can also be increased or attenuated by introducing mutations into the coding sequence of a gene or into non-coding sequences. These mutations may be synonymous, when no modification in the corresponding amino acid occurs, or nonsynonymous, when the corresponding amino acid is altered. Synonymous mutations do not have any impact on the function of translated proteins, but may have an impact on the regulation of the corresponding genes or even of other genes, if the mutated sequence is located in a binding site for a regulator factor. Non-synonymous mutations may have an impact on the function or activity of the translated protein as well as on regulation, depending on the nature of the mutated sequence.

In particular, mutations in non-coding sequences may be located upstream of the coding sequence (i.e., in the promoter region, in an enhancer, silencer, or insulator region, in a specific transcription factor binding site) or downstream of the coding sequence. Mutations introduced in the promoter region may be in the core promoter, proximal promoter or distal promoter. Mutations may be introduced by site-directed mutagenesis using, for example, Polymerase Chain Reaction (PCR), by random mutagenesis techniques for example via mutagenic agents (Ultra-Violet rays or chemical agents like nitrosoguanidine (NTG) or ethylmethanesulfonate (EMS)) or DNA shuffling or error-prone PCR or using culture conditions that apply a specific stress on the microorganism and induce mutagenesis. The insertion of one or more supplementary nucleotide(s) in the region located upstream of a gene can notably modulate gene expression.

A particular way of modulating endogenous gene expression is to exchange the endogenous promoter of a gene (e.g., wild-type promoter) with a stronger or weaker promoter to upregulate or downregulate expression of the endogenous gene. The promoter may be endogenous (i.e., originating from the same species) or exogenous (i.e., originating from a different species). It is well within the ability of the person skilled in the art to select an appropriate promoter for modulating the expression of an endogenous gene. Such a promoter be, for example, a Ptrc, Ptac, Ptet, or Plac promoter, or a lambda P_{L} (PL) or lambda P_{R} (PR) promoter. The promoter may be "inducible" by a particular compound or by specific external conditions, such as temperature or light or a small molecule, such as an antibiotic.

A particular way of modulating endogenous protein activity is to introduce nonsynonymous mutations in the coding sequence of the corresponding gene, e.g., according to any of the methods described above. A non-synonymous amino acid mutation that is present in a transcription factor may notably alter binding affinity of the transcription factor toward a cis-element, alter ligand binding to the transcription factor, etc.

A microorganism may also be genetically modified to express one or more exogenous or heterologous genes so as to overexpress the corresponding gene product (e.g., an enzyme). An "exogenous" or "heterologous" gene as used herein refers to a gene encoding a protein or polypeptide that is introduced into a microorganism in which said gene does not naturally occur. In particular, a heterologous gene may be directly integrated into the chromosome of the microorganism, or be expressed extra-chromosomally within the microorganism by plasmids or vectors. For successful expression, the heterologous gene(s) must be introduced into the microorganism with all of the regulatory elements necessary for their expression or be introduced into a microorganism that already comprises all of the regulatory elements necessary for their expression. The genetic modification or transformation of microorganisms with one or more exogenous genes is a routine task for those skilled in the art.

One or more copies of a given heterologous gene can be introduced on a chromosome by methods well-known in the art, such as by genetic recombination. When a gene is expressed extra-chromosomally, it can be carried by a plasmid or a vector. Different types of plasmids are notably available, which may differ in respect to their origin of replication and/or on their copy number in the cell.

On the basis of a given amino acid sequence, the skilled person is furthermore able to identify an appropriate polynucleotide coding for said polypeptide (e.g., in the available databases, such as Uniprot), or to synthesize the corresponding polypeptide or a polynucleotide coding for said polypeptide. De novo synthesis of a polynucleotide can be performed, for example, by initially synthesizing individual nucleic acid oligonucleotides and hybridizing these with oligonucleotides complementary thereto, such that they form a double-stranded DNA molecule, and then ligating the individual double-stranded oligonucleotides such that the desired nucleic acid sequence is obtained.

The terms "enhanced", "expressing," "overexpressing," or "overexpression" of a protein of interest, such as an enzyme, refer herein to a significant increase in the expression level and/or activity of said protein in a microorganism, as compared to the parent microorganism in which the expression level and/or activity of said protein of interest is not modified, especially not genetically modified. In contrast, the terms "attenuated", "attenuating" or "attenuation" of a protein of interest refer to a significant decrease in the expression level and/or activity of said protein in a microorganism, as compared to the parent microorganism in which the expression level and/or activity of said protein of interest is not modified, especially not genetically modified. The complete attenuation of the expression level and/or activity of a protein of interest means that expression and/or activity is abolished; thus, the expression level of said protein is null. Where the "enhanced", "expressing," "overexpressing,", "overexpression" "attenuated", "attenuating" or "attenuation" is/are due to a mutation in the corresponding gene or protein of interest, the modification of the expression level and/or activity of said gene or protein of interest is compared to the expression level and/or activity of the same non-mutated gene or protein of interest.

The terms "production," "overproducing," or "overproduction" of a protein of interest, such as an enzyme, refer herein to an increase in the production level and/or activity of said protein in a microorganism, as compared to the corresponding parent microorganism that does not comprise the modification present in the genetically modified microorganism (i.e., in the unmodified microorganism). A heterologous gene or protein can be considered to be respectively "expressed" or "overexpressed" and "produced" or "overproduced" in a genetically modified microorganism when compared with a corresponding parent microorganism in which said heterologous gene or protein is absent. In contrast, the terms "attenuating" or "attenuation" of the synthesis of a protein of interest refer to a decrease in the production level and/or activity of said protein in a microorganism, as compared to the parent microorganism. Similarly, an "attenuation" of gene expression refers to a decrease in the level of gene expression as compared to the parent microorganism. An attenuation of expression can notably be due to either the exchange of the wild-type promoter for a weaker natural or synthetic promoter or the use of an agent reducing gene expression, such as antisense RNA or interfering RNA (RNAi), and more particularly small interfering RNAs (siRNAs) or short hairpin RNAs (shRNAs). Promoter exchange may notably be achieved by the technique of homologous recombination (Datsenko & Wanner, 2000). The complete attenuation of the production level and/or activity of a protein of interest means that production and/or activity is abolished; thus, the production level of said protein is null. The complete attenuation of the production level and/or activity of a protein of interest may be due to the complete suppression of the expression of a gene. This suppression can be either an inhibition of the expression of the gene, a deletion of all or part of the promoter region necessary for expression of the gene, or a deletion of all or part of the coding region of the gene. A deleted gene can notably be replaced by a selection marker gene that facilitates the identification, isolation and purification of the modified microorganism. As a non-limiting example, suppression of gene expression may be achieved by the technique of homologous recombination, which is well-known to the person skilled in the art (Datsenko & Wanner, 2000).

The activity of each enzyme mentioned herein which is modified in the *Clostridium* mutant strain of the invention is intended to mean the specific activity.

In addition, the activity of each enzyme which is modified in the *Clostridium* mutant strain in the context of the invention is defined compared to a non-mutated strain or wild-type *Clostridium* strain, in particular of *Clostridium acetobutylicum.*

Modulating the production level of one or more proteins may thus occur by altering the expression of one or more endogenous genes that encode said protein within the microorganism as described above and/or by introducing one or more heterologous genes that encode said protein(s) into the microorganism.

The term "production level" or "expression level" as used herein, refers to the amount (e.g., relative amount, concentration) of a protein of interest (or of the gene encoding said protein) expressed in a microorganism, which is measurable by methods well-known in the art. The level of gene expression can be measured by various known methods including Northern blotting, quantitative RT-PCR, and the like. Alternatively, the level of production of the protein coded by said gene may be measured, for example by SDS-PAGE, HPLC:LC/MS and other quantitative proteomic techniques (Bantscheff et al., 2007), or, when antibodies against said protein are available, by Western Blot-Immunoblot (Burnette, 1981), Enzyme-linked immunosorbent assay (e.g., ELISA) (Engvall and Perlman, 1971), protein immunoprecipitation, immunoelectrophoresis, and the like. The copy number of an expressed gene can be quantified, for example, by restricting chromosomal DNA followed by Southern blotting using a probe based on the gene sequence, fluorescence in situ hybridization (FISH), qPCR, and the like.

Overexpression of a given gene or overproduction of the corresponding protein may be verified by comparing the expression level of said gene or the level of synthesis of said protein in the genetically modified organism to the expression level of the same gene or the level of synthesis of the same protein, respectively, in a control microorganism that does not have the genetic modification (i.e., the parental strain or unmodified microorganism).

In the context of the present invention, the method for the production of glycerol and acetate comprising culturing, in an appropriate culture medium, a *Clostridium* mutant strain comprising an overexpression of at least one of glycerol phosphate phosphatase and glycerol phosphate dehydrogenase activity.

Preferably, the method for the production of glycerol and acetate comprising culturing, in an appropriate culture medium, a *Clostridium* mutant strain comprising an overexpression of glycerol phosphate phosphatase and glycerol phosphate dehydrogenase activity.

Particularly, the method according to the present invention comprises culturing a mutant strain wherein the glycerol phosphate phosphatase is encoded by the gene *CA_C1625* at the locus 1764289 in the *C*. *acetobutylicum* genome ATCC824, Genbank AE001437. Exemplary gene and amino acid sequences have at least 80%, 90%, 95%, or 100% sequence identity with the sequences are shown in SEQ ID NOs: 1 and 2, respectively.

Similarly, the method according to the present invention comprises culturing a mutant strain wherein the glycerol phosphate dehydrogenase is preferably encoded by the gene *CA_C1626* at the locus 1764945 in the *C*. *acetobutylicum* genome ATCC824, Genbank AE001437. Exemplary gene and amino acid sequences have at least 80%, 90%, 95%, or 100% sequence identity with the sequences are shown in SEQ ID NOs: 3 and 4, respectively.

In the context of the present invention, overexpression may result from an increased expression of endogenous gene or expression of exogenous gene. A person skilled in the art can easily induce the overexpression of a gene or protein of interest in a microorganism. In particular, gene editing tools may be used, for example the CRISPR/Cas9 tool, particularly as demonstrated by Wilding-Steele *et al.* (2021).

In the context of the method of the invention, the mutant strain may comprise additional modifications with regards to the parental un-modified strain.

In a particular embodiment, the method of the invention comprises culturing a *Clostridium* mutant strain as described above and wherein at least one gene encoding an enzyme involved in production of metabolites from glycerol is attenuated, preferably deleted.

Preferably, in the method of the invention, the *Clostridium* mutant strain further comprises an attenuation of the butyrate formation, and more preferably an inhibition of the butyrate formation, as compared to an unmodified microorganism. In particular, in the method of the invention, the *Clostridium* mutant strain also comprises an attenuation, preferably an inhibition of the phospho-transbutyrylase and/or butyrate kinase activities, as compared to an unmodified microorganism.

Most preferably, the microorganism comprises an attenuated Ptb and/or Buk activity.

Preferably, the Ptb and Buk proteins have at least 80%, 90%, 95%, or 100% sequence similarity or sequence identity with the sequence of SEQ ID NOs: 6 and 8, respectively.

Preferably, the attenuation of said proteins results from an attenuation of at least one of the genes coding said proteins (i.e., *ptb* and/or *buk* genes). Preferably, the *ptb* and *buk* genes have at least 80%, 90%, 95%, or 100% sequence identity with the sequences of SEQ ID NOs: 5 and 7, respectively.

Preferably, expression of Ptb and/or Buk is partially or completely attenuated. Preferably, attenuation of Ptb and/or Buk activity results from an inhibition of expression of the *ptb* and/or *buk* genes coding said enzymes. Preferably, attenuation of expression results from a partial or complete deletion of the *ptb* and/or *buk* genes.

Most preferably, in the method of the invention, the *Clostridium* mutant strain comprises a deletion of the *ptb* and *buk* genes.

Preferably, in the method of the invention, the *Clostridium* mutant strain further comprises an attenuation of the lactate formation, and more preferably an inhibition of the lactate formation, as compared to an unmodified microorganism. In particular, in the method of the invention, the *Clostridium* mutant strain also comprises an attenuation, preferably an inhibition of the lactate dehydrogenase activity, as compared to an unmodified microorganism.

Most preferably, the microorganism comprises an attenuated Ldh activity.

Preferably, the Ldh protein have at least 80%, 90%, 95%, or 100% sequence similarity or sequence identity with the sequence of SEQ ID NO: 10.

Preferably, the attenuation of said protein results from an attenuation of the gene coding said protein (i.e., *ldh* gene). Preferably, the *ldh* gene have at least 80%, 90%, 95%, or 100% sequence identity with the sequences of SEQ ID NO: 9.

Preferably, expression of Ldh is partially or completely attenuated. Preferably, attenuation of Ldh activity results from an inhibition of expression of the *ldh* gene coding said enzyme. Preferably, attenuation of expression results from a partial or complete deletion of the *ldh* gene.

Most preferably, in the method of the invention, the *Clostridium* mutant strain comprises a deletion of the *ldh* gene.

Preferably, in the method of the invention, the *Clostridium* mutant strain further comprises an attenuation of the ethanol and butanol formation, and more preferably an inhibition of the ethanol and butanol formation, as compared to an unmodified microorganism. In particular, in the method of the invention, the *Clostridium* mutant strain also comprises an attenuation, preferably an inhibition of the aldehyde-alcohol dehydrogenase activity, as compared to an unmodified microorganism.

Most preferably, the microorganism comprises an attenuated AdhE1 and/or AdhE2 activity.

Preferably, the AdhE1 and AdhE2 proteins have at least 80%, 90%, 95%, or 100% sequence similarity or sequence identity with the sequence of SEQ ID NOs: 12 and 14, respectively.

Preferably, the attenuation of said proteins results from an attenuation of at least one of the genes coding said proteins (i.e., *adhE1* and/or *adhE2* genes). Preferably, the *adhE1* and *adhE2* genes have at least 80%, 90%, 95%, or 100% sequence identity with the sequences of SEQ ID NOs: 11 and 13, respectively.

Preferably, expression of AdhE1 and/or AdhE2 is partially or completely attenuated. Preferably, attenuation of AdhE1 and/or AdhE2 activity results from an inhibition of expression of the *adhE1* and/or *adhE2* genes coding said enzymes. Preferably, attenuation of expression results from a partial or complete deletion of the *adhE1* and/or *adhE2* genes, more preferably from a partial or complete deletion of the *adhE1* and *adhE2* genes.

Most preferably, in the method of the invention, the *Clostridium* mutant strain comprises a deletion of the *adhE1* and *adhE2* genes.

Preferably, the method of the invention comprises culturing a *Clostridium* mutant strain wherein the *adhE1* and *adhE2* genes are deleted by curing the *Clostridium* mutant strain for the pSOL1 megaplasmid.

In particular, the method of the invention comprises culturing a *Clostridium* mutant strain wherein at least one gene chosen among the following, and as defined above, is attenuated, preferably deleted:
- *ptb* gene encoding phospho-transbutyrylase to avoid butyrate formation,
- *buk* gene encoding butyrate kinase to avoid butyrate formation,
- *ldh* gene encoding lactate dehydrogenase to avoid lactate formation,
- *adhE1* and *adhE2* genes encoding aldehyde-alcohol dehydrogenase to avoid ethanol and butanol formation.

Preferably, the method of the invention comprises culturing a *Clostridium* mutant strain wherein the expression of the following genes, and as defined above, is attenuated, preferably deleted:
- *ptb* gene encoding phospho-transbutyrylase to avoid butyrate formation,
- *buk* gene encoding butyrate kinase to avoid butyrate formation,
- *ldh* gene encoding lactate dehydrogenase to avoid lactate formation, and
- *adhE1* and *adhE2* genes encoding aldehyde-alcohol dehydrogenase to avoid ethanol and butanol formation.

In a particular embodiment, the method of the invention comprises culturing a *Clostridium* mutant strain as described above and which convert acetate to acetone.

Preferably, in the method of the invention, the *Clostridium* mutant strain further comprises an increased CoA-transferase activity, as compared to an unmodified microorganism. It is preferably an increased CoA-transferase subunit A and/or CoA-transferase subunit B activity.

Preferably, the CtfA and CtfB proteins have at least 80%, 90%, 95%, or 100% sequence similarity or sequence identity with the sequence of SEQ ID NOs: 16 and 18, respectively. The CtfA and/or CtfB enzyme may be from a solventogenic *Clostridium* genus. Preferably CtfA and/or CtfB enzyme is from *Clostridium beijerinckii.*

CtfA and CtfB preferably have at least 80%, 90%, 95%, or 100% sequence similarity or sequence identity with the CtfA and CtfB enzymes having the sequence of SEQ ID NOs: 16 or 18. CtfA and/or CtfB may be a functional variant or functional fragment of one of the CtfA and CtfB enzymes described herein.

A "functional variant" of an enzyme as described herein includes, but is not limited to, enzymes having amino acid sequences which are at least 60% similar or identical after alignment to the amino acid sequence encoding an enzyme as provided herein. According to the present invention, such a variant preferably has at least 70%, 80%, 85%, 90%, 95%, 96%, 97%, 98%, 99%, or 99.9% amino acid sequence similarity or identity to the protein described herein. Said functional variant furthermore has the same enzymatic function as the enzyme provided herein. As a non-limiting example, a functional variant of CtfA or CtfB of SEQ ID NOs: 16 or 18 has at least 70%, 80%, 85%, 90%, 95%, 96%, 97%, 98%, 99%, or 99.9% sequence identity to said respective sequences. As a non-limiting example, means of determining sequence identity are further provided herein.

Preferably, the activity of the CtfA or CtfB enzyme is due to an overexpression of the gene coding for the enzyme. The corresponding *ctfA* and *ctfB* gene, which codes CtfA and CtfB, preferably has at least 80%, 90%, 95%, or 100% sequence identity with SEQ ID NOs: 15 or 17.

Preferably, in the method of the invention, the *Clostridium* mutant strain further comprises an increased activity of proteins CtfA and CtfB from *Clostridium beijerinckii,* as compared to an unmodified microorganism, more preferably due to an overexpression of the corresponding *ctfA* and *ctfB* genes from *Clostridium beijerinckii.*

Preferably, in the method of the invention, the *Clostridium* mutant strain further comprises an increased CoA acylating aldehyde dehydrogenase activity, as compared to an unmodified microorganism. It is preferably an increased Adc activity.

Preferably, the Adc protein have at least 80%, 90%, 95%, or 100% sequence similarity or sequence identity with the sequence of SEQ ID NO: 20. The ADC enzyme may be from a solventogenic *Clostridium* genus. Preferably the Adc enzyme is from *Clostridium beijerinckii.*

ADC preferably has at least 80%, 90%, 95%, or 100% sequence similarity or sequence identity with the Adc enzyme having the sequence of SEQ ID NO: 20. ADC may be a functional variant or functional fragment of one of the Adc enzymes described herein.

A "functional variant" of an enzyme as described herein includes, but is not limited to, enzymes having amino acid sequences which are at least 60% similar or identical after alignment to the amino acid sequence encoding an enzyme as provided herein. According to the present invention, such a variant preferably has at least 70%, 80%, 85%, 90%, 95%, 96%, 97%, 98%, 99%, or 99.9% amino acid sequence similarity or identity to the protein described herein. Said functional variant furthermore has the same enzymatic function as the enzyme provided herein. As a non-limiting example, a functional variant of Adc of SEQ ID NO: 20 has at least 70%, 80%, 85%, 90%, 95%, 96%, 97%, 98%, 99%, or 99.9% sequence identity to said respective sequences. As a non-limiting example, means of determining sequence identity are further provided herein.

Preferably, the activity of the Adc enzyme is due to an overexpression of the gene coding for the enzyme. The corresponding adc gene, which codes Adc, preferably has at least 80%, 90%, 95%, or 100% sequence identity with SEQ ID NO: 19.

Preferably, in the method of the invention, the *Clostridium* mutant strain further comprises an increased activity of protein Adc from *Clostridium beijerinckii,* as compared to an unmodified microorganism, more preferably due to an overexpression of the corresponding adc gene from *Clostridium beijerinckii.*

In a particular embodiment, the method of the invention comprises culturing a *Clostridium* mutant strain which is modified to convert acetate to acetone by overexpressing at least one gene selected among:
- *ctfA* gene from a solventogenic Clostridium and encoding CoA-transferase subunit A, preferably from *Clostridium beijerinckii,*
- *ctfB* gene from a solventogenic *Clostridium* and encoding CoA-transferase subunit B, preferably from *Clostridium beijerinckii,*
- adc gene from a solventogenic *Clostridium* and encoding coenzyme A-acylating aldehyde dehydrogenase, preferably from *Clostridium beijerinckii.*

In a particular embodiment, the method of the invention comprises culturing a *Clostridium* mutant strain which is modified to convert acetate to acetone by overexpressing the following genes:
- *ctfA* gene from a solventogenic Clostridium and encoding CoA-transferase subunit A, preferably from *Clostridium beijerinckii,*
- *ctfB* gene from a solventogenic *Clostridium* and encoding CoA-transferase subunit B, preferably from *Clostridium beijerinckii,* and
- adc gene from a solventogenic *Clostridium* and encoding coenzyme A-acylating aldehyde dehydrogenase, preferably from *Clostridium beijerinckii.*

Preferably, in the method of the invention, the *Clostridium* mutant strain further comprises an attenuation of the 3-hydroxybutyryl-CoA dehydrogenase activity, and more preferably an inhibition of the 3-hydroxybutyryl-CoA dehydrogenase activity, as compared to an unmodified microorganism. Most preferably, the mutant strain comprises an attenuated Hbd activity.

Preferably, the Hbd protein has at least 80%, 90%, 95%, or 100% sequence similarity or sequence identity with the sequence of SEQ ID NO: 22.

Preferably, the attenuation of said protein results from an attenuation of the gene coding said protein (i.e., *hbd* gene). Preferably, the *hbd* gene has at least 80%, 90%, 95%, or 100% sequence identity with the sequences of SEQ ID NO: 21.

Preferably, expression of Hbd is partially or completely attenuated. Preferably, attenuation of Hbd activity results from an inhibition of expression of the *hbd* gene coding said enzyme. Preferably, attenuation of expression results from a partial or complete deletion of the *hbd* gene.

More preferably, the *hbd* gene encoding 3-hydroxybutyryl-CoA dehydrogenase is also deleted in the *Clostridium* mutant strain.

In a particular embodiment, the method of the invention comprises culturing a *Clostridium* mutant strain which is modified to produce 1,3-propanediol from glycerol.

Preferably, in the method of the invention, the *Clostridium* mutant strain further comprises an increased B12-independent glycerol dehydratase activity, as compared to an unmodified microorganism. It is preferably an increased DhaB1 activity.

Preferably, the DhaB1 protein have at least 80%, 90%, 95%, or 100% sequence similarity or sequence identity with the sequence of SEQ ID NO: 24. The DhaB1 enzyme may be from a species of the *Clostridium* genus. Preferably the DhaB1 enzyme is from *Clostridium butyricum.*

DhaB1 preferably has at least 80%, 90%, 95%, or 100% sequence similarity or sequence identity with the DhaB1 enzyme having the sequence of SEQ ID NO: 24. DhaB1 may be a functional variant or functional fragment of one of the DhaB1 enzymes described herein.

A "functional A variant" of an enzyme as described herein includes, but is not limited to, enzymes having amino acid sequences which are at least 60% similar or identical after alignment to the amino acid sequence encoding an enzyme as provided herein. According to the present invention, such a variant preferably has at least 70%, 80%, 85%, 90%, 95%, 96%, 97%, 98%, 99%, or 99.9% amino acid sequence similarity or identity to the protein described herein. Said functional variant furthermore has the same enzymatic function as the enzyme provided herein. As a non-limiting example, a functional variant of DhaB1 of SEQ ID NO: 24 has at least 70%, 80%, 85%, 90%, 95%, 96%, 97%, 98%, 99%, or 99.9% sequence identity to said respective sequences. As a non-limiting example, means of determining sequence identity are further provided herein.

Preferably, the activity of the DhaB1enzyme is due to an overexpression of the gene coding for the enzyme. The corresponding *dhaB1* gene, which codes DhaB1, preferably has at least 80%, 90%, 95%, or 100% sequence identity with SEQ ID NO: 23.

Preferably, in the method of the invention, the Clostridium mutant strain further comprises an increased activity of protein DhaB1from *Clostridium butyricum,* as compared to an unmodified microorganism, more preferably due to an overexpression of the corresponding *dhaB1* gene from *Clostridium butyricum.*

Preferably, in the method of the invention, the Clostridium mutant strain further comprises an increased glycerol dehydratase activator activity, as compared to an unmodified microorganism. It is preferably an increased DhaB2 activity.

Preferably, the DhaB2 protein have at least 80%, 90%, 95%, or 100% sequence similarity or sequence identity with the sequence of SEQ ID NO: 26. The DhaB2 enzyme may be from a species of the *Clostridium* genus. Preferably the DhaB2 enzyme is from *Clostridium butyricum.*

DhaB2 preferably has at least 80%, 90%, 95%, or 100% sequence similarity or sequence identity with the DhaB2 enzyme having the sequence of SEQ ID NO: 26. DhaB2 may be a functional variant or functional fragment of one of the DhaB2 enzymes described herein.

A "functional variant" of an enzyme as described herein includes, but is not limited to, enzymes having amino acid sequences which are at least 60% similar or identical after alignment to the amino acid sequence encoding an enzyme as provided herein. According to the present invention, such a variant preferably has at least 70%, 80%, 85%, 90%, 95%, 96%, 97%, 98%, 99%, or 99.9% amino acid sequence similarity or identity to the protein described herein. Said functional variant furthermore has the same enzymatic function as the enzyme provided herein. As a non-limiting example, a functional variant of DhaB2 of SEQ ID NO: 26 has at least 70%, 80%, 85%, 90%, 95%, 96%, 97%, 98%, 99%, or 99.9% sequence identity to said respective sequences. As a non-limiting example, means of determining sequence identity are further provided herein.

Preferably, the activity of the DhaB2 enzyme is due to an overexpression of the gene coding for the enzyme. The corresponding *dhaB2* gene, which codes DhaB2, preferably has at least 80%, 90%, 95%, or 100% sequence identity with SEQ ID NO: 25.

Preferably, in the method of the invention, the *Clostridium* mutant strain further comprises an increased activity of protein DhaB2 from *Clostridium butyricum,* as compared to an unmodified microorganism, more preferably due to an overexpression of the corresponding *dhaB2* gene from *Clostridium butyricum.*

Preferably, in the method of the invention, the *Clostridium* mutant strain further comprises an increased 1,3-propanediol dehydrogenase activity or both NADP+ dependent alcohol dehydrogenase and Fd-NADP+ reductase increased activities, as compared to an unmodified microorganism.

Preferably, the increased 1,3-propanediol dehydrogenase activity is an increased DhaT activity.

Preferably, the DhaT protein have at least 80%, 90%, 95%, or 100% sequence similarity or sequence identity with the sequence of SEQ ID NO: 28. The DhaT enzyme may be from a species of the *Clostridium* genus. Preferably the DhaT enzyme is from *Clostridium butyricum.*

DhaT preferably has at least 80%, 90%, 95%, or 100% sequence similarity or sequence identity with the DhaT enzyme having the sequence of SEQ ID NO: 28. DhaT may be a functional variant or functional fragment of one of the DhaT enzymes described herein.

A "functional variant" of an enzyme as described herein includes, but is not limited to, enzymes having amino acid sequences which are at least 60% similar or identical after alignment to the amino acid sequence encoding an enzyme as provided herein. According to the present invention, such a variant preferably has at least 70%, 80%, 85%, 90%, 95%, 96%, 97%, 98%, 99%, or 99.9% amino acid sequence similarity or identity to the protein described herein. Said functional variant furthermore has the same enzymatic function as the enzyme provided herein. As a non-limiting example, a functional variant of DhaT of SEQ ID NO: 28 has at least 70%, 80%, 85%, 90%, 95%, 96%, 97%, 98%, 99%, or 99.9% sequence identity to said respective sequences. As a non-limiting example, means of determining sequence identity are further provided herein.

Preferably, the activity of the DhaT enzyme is due to an overexpression of the gene coding for the enzyme The corresponding *dhaT* gene, which codes DhaT, preferably has at least 80%, 90%, 95%, or 100% sequence identity with SEQ ID NO: 27.

Preferably, in the method of the invention, the *Clostridium* mutant strain further comprises an increased activity of protein DhaT from *Clostridium butyricum,* as compared to an unmodified microorganism, more preferably due to an overexpression of the corresponding *dhaT* gene from *Clostridium butyricum.*

Preferably, the NADP+ dependent alcohol dehydrogenase increased activity is a BdhB increased activity.

Preferably, the BdhB protein have at least 80%, 90%, 95%, or 100% sequence similarity or sequence identity with the sequence of SEQ ID NO: 37. The BdhB enzyme may be from a species of the *Clostridium* genus. Preferably the BdhB enzyme is from *Clostridium acetobutylicum.*

BdhB preferably has at least 80%, 90%, 95%, or 100% sequence similarity or sequence identity with the BdhB enzyme having the sequence of SEQ ID NO: 37. BdhB may be a functional variant or functional fragment of one of the BdhB enzymes described herein.

A "functional variant" of an enzyme as described herein includes, but is not limited to, enzymes having amino acid sequences which are at least 60% similar or identical after alignment to the amino acid sequence encoding an enzyme as provided herein. According to the present invention, such a variant preferably has at least 70%, 80%, 85%, 90%, 95%, 96%, 97%, 98%, 99%, or 99.9% amino acid sequence similarity or identity to the protein described herein. Said functional variant furthermore has the same enzymatic function as the enzyme provided herein. As a non-limiting example, a functional variant of BdhB of SEQ ID NO: 37 has at least 70%, 80%, 85%, 90%, 95%, 96%, 97%, 98%, 99%, or 99.9% sequence identity to said respective sequences. As a non-limiting example, means of determining sequence identity are further provided herein.

Preferably, the activity of the BdhB enzyme is due to an overexpression of the gene coding for the enzyme The corresponding *bdhb* gene, which codes BdhB, preferably has at least 80%, 90%, 95%, or 100% sequence identity with SEQ ID NO: 36.

Preferably, in the method of the invention, the Clostridium mutant strain further comprises an increased activity of protein BdhB from *Clostridium acetobutylicum,* as compared to an unmodified microorganism, more preferably due to an overexpression of the corresponding *bdhb* gene from *Clostridium acetobutylicum.*

Preferably, the Fd-NADP+ reductase has at least 80%, 90%, 95%, or 100% sequence similarity or sequence identity with the sequence of SEQ ID NO: 39. This enzyme may be from a species of the *Clostridium* genus, preferably from *Clostridium acetobutylicum.*

The Fd-NADP+ reductase preferably has at least 80%, 90%, 95%, or 100% sequence similarity or sequence identity with the Fd-NADP+ reductase having the sequence of SEQ ID NO: 39. It may be a functional variant or functional fragment of one of the Fd-NADP+ reductases described herein.

A "functional A variant" of an enzyme as described herein includes, but is not limited to, enzymes having amino acid sequences which are at least 60% similar or identical after alignment to the amino acid sequence encoding an enzyme as provided herein. According to the present invention, such a variant preferably has at least 70%, 80%, 85%, 90%, 95%, 96%, 97%, 98%, 99%, or 99.9% amino acid sequence similarity or identity to the protein described herein. Said functional variant furthermore has the same enzymatic function as the enzyme provided herein. As a non-limiting example, a functional variant of the Fd-NADP+ reductase of SEQ ID NO: 39 has at least 70%, 80%, 85%, 90%, 95%, 96%, 97%, 98%, 99%, or 99.9% sequence identity to said respective sequences. As a non-limiting example, means of determining sequence identity are further provided herein.

Preferably, the activity of the Fd-NADP+ reductase is due to an overexpression of the gene coding for the enzyme. The corresponding *CA_C0764* gene, which codes the Fd-NADP+ reductase, preferably has at least 80%, 90%, 95%, or 100% sequence identity with SEQ ID NO: 38.

Preferably, in the method of the invention, the *Clostridium* mutant strain further comprises an increased activity of Fd-NADP+ reductase from *Clostridium acetobutylicum,* as compared to an unmodified microorganism, more preferably due to an overexpression of the corresponding *CA_C0764* gene from *Clostridium acetobutylicum.*

In a particular embodiment, the method of the invention comprises culturing a *Clostridium* mutant strain which is modified to produce 1,3-propanediol from glycerol by overexpressing at least one of the following genes:
- *dhaB1* gene from *Clostridium butyricum* and encoding B12-independent glycerol dehydratase,
- *dhaB2* gene from *Clostridium butyricum* and encoding glycerol dehydratase activator,
- either *dhaT* gene from *Clostridium butyricum* and encoding 1,3-propanediol dehydrogenase or both *bdhb* gene from *Clostridium acetobutylicum* encoding an NADP+ dependent alcohol dehydrogenase and *CA_C0764* gene from *Clostridium acetobutylicum* encoding a Fd-NADP+ reductase.

In a particular embodiment, the method of the invention comprises culturing a *Clostridium* mutant strain which is modified to produce 1,3-propanediol from glycerol by overexpressing the following genes:
- *dhaB1* gene from *Clostridium butyricum* and encoding B12-independent glycerol dehydratase,
- *dhaB2* gene from *Clostridium butyricum* and encoding glycerol dehydratase activator, and
- *dhaT* gene from *Clostridium butyricum* and encoding 1,3-propanediol dehydrogenase.

In a particular embodiment, the method of the invention comprises culturing a *Clostridium* mutant strain which is modified to produce 1,3-propanediol from glycerol by overexpressing the following genes:
- *dhaB1* gene from *Clostridium butyricum* and encoding B12-independent glycerol dehydratase,
- *dhaB2* gene from *Clostridium butyricum* and encoding glycerol dehydratase activator, and
- *bdhb* gene from *Clostridium acetobutylicum* encoding an NADP+ dependent alcohol dehydrogenase and *CA_C0764* gene from *Clostridium acetobutylicum* encoding a Fd-NADP+ reductase.

Preferably, in the method of the invention, the *Clostridium* mutant strain further comprises an attenuation of the hydrogenase I activity, and more preferably an inhibition of the HydA activity, as compared to an unmodified microorganism.

Preferably, the HydA protein has at least 80%, 90%, 95%, or 100% sequence similarity or sequence identity with the sequence of SEQ ID NO: 30.

Preferably, the attenuation of said protein results from an attenuation of the gene coding said protein (i.e., *hydA* gene). Preferably, the *hydA* gene has at least 80%, 90%, 95%, or 100% sequence identity with the sequences of SEQ ID NO: 29.

Preferably, expression of HydA is partially or completely attenuated. Preferably, attenuation of HydA activity results from an inhibition of expression of the *hydA* gene coding said enzyme. Preferably, attenuation of expression results from a partial or complete deletion of the *hydA* gene.

Preferably, the *hydA* gene encoding hydrogenase I is attenuated in the *Clostridium* mutant strain, more preferably deleted.

In a more particular embodiment, the method of the invention comprises culturing a *Clostridium* mutant strain which, in addition to an overexpression of at least one of glycerol phosphate phosphatase and glycerol phosphate dehydrogenase activity, is further modified to:
- attenuate production of metabolites from glycerol as described herein, and/or
- convert acetate to acetone as described herein, and/or
- to produce 1,3-propanediol from glycerol as described herein.

Preferably, the method of the invention comprises culturing a *Clostridium* mutant strain which, in addition to an overexpression of at least one of glycerol phosphate phosphatase and glycerol phosphate dehydrogenase activity, is further modified to attenuate production of metabolites from glycerol as described herein, and:
- convert acetate to acetone as described herein, or
- to produce 1,3-propanediol from glycerol as described herein.

In an advantageous embodiment, the method of the invention comprises culturing a *Clostridium* mutant strain which, in addition to an overexpression of at least one of glycerol phosphate phosphatase and glycerol phosphate dehydrogenase activity, is further modified to attenuate production of metabolites from glycerol as described herein, and:
- convert acetate to acetone as described herein, and
- to produce 1,3-propanediol from glycerol as described herein.

In a particular embodiment, the method of the invention comprises culturing a *Clostridium* mutant strain, as described herein, selected from the group consisting of *Clostridium acetobutylicum, Clostridium butyricum, Clostridium beijerinckii, Clostridium pasteurianum and Clostridium thermocellum.* Preferably, the method of the invention comprises culturing a *Clostridium acetobutylicum* mutant strain.

The "appropriate culture medium" or a "culture medium" refers to a culture medium appropriate for the growth of the *Clostridium* mutant strain and the synthesis of product of interest by the cells, and in particular the diol-production. Preferably, the source of carbon in the culture medium comprises or consist in glucose, fructose, xylose and/or sucrose. More preferably, the source of carbon in the culture medium comprises at least glucose. In the culture medium of the present invention, glucose is advantageously the single source of carbon. The culture medium may or may not comprise an organic nitrogen source. Nitrogen is a naturally occurring element that is essential for growth and reproduction in both plants and animals. It is found in amino acids and in many other organic and inorganic compounds. "Organic nitrogen" means, according to the invention, a nitrogen comprising organic compound obtained from living organisms. Usual sources of organic nitrogen for bacterial culture comprise yeast extract.

The fermentation process is generally conducted in reactors with a synthetic, particularly inorganic, culture medium of known defined composition adapted to the *Clostridium* species used and containing at least glucose.

The man skilled in the art knows how to manage each of the experimental conditions, and to define the culture conditions for the *Clostridia* strains used according to the invention. In particular *Clostridia* strains are fermented at a temperature between 20°C and 60°C, preferentially between 25°C and 40°C for C. *acetobutylicum.*

In the method of the invention, the production is advantageously done in a batch, fed-batch or continuous process. Culturing *Clostridium* microorganisms at industrial scale for the production of glycerol, acetate, acetone, and/or PDO is known in the art, for example as disclosed in WO2010/128070 and WO2011/042434, which content are incorporated herein by reference.

Preferably, the method according to the invention further comprises recovering the PDO and/or acetone produced from the culture medium, and optionally may be purified. Methods for recovering and eventually purifying PDO from a fermentation medium are known to the skilled person. PDO may be isolated by distillation. A particular purification method is disclosed in applications WO2009/068110 and WO2010/037843, which content is incorporated herein by reference.

In a second aspect, the present invention concerns a mutant strain of *Clostridium* with improved glycerol and acetate production capacities, comprising an overexpression of at least one of glycerol phosphate phosphatase encoded by the gene *CA_C1625* at the locus 1764289 in the *C*. *acetobutylicum* genome ATCC824, Genbank AE001437 with SEQ ID NO: 1 and glycerol phosphate dehydrogenase encoded by the gene *CA_C1626* at the locus 1764945 in the *C*. *acetobutylicum* genome ATCC824, Genbank AE001437 with SEQ ID NO: 3, preferably both.

In the context of the present invention, the *Clostridium* mutant strain may further comprise an overexpression of at least one of glycerol phosphate phosphatase and glycerol phosphate dehydrogenase activity.

Preferably, in the *Clostridium* mutant strain of the invention at least one gene encoding an enzyme involved in production of metabolites from glycerol is attenuated, preferably deleted.

In a more preferred embodiment, the *Clostridium* mutant strain is modified to convert acetate to acetone and/or to produce 1,3-propanediol from glycerol.

All the preferred embodiments for the *Clostridium* mutant strain as described above with relation to the method of the invention for producing glycerol and acetate, especially where combined with any other additional genetic modifications leading to conversion of acetate in acetone and PDO production capacities, apply *mutatis mutandis* to the mutant strain of *Clostridium* according to the second aspect of the present invention.

### FIGURES

**Figure 1** represents the consumption of glucose and the production of glycerol, acetate acetone and PDO, by culturing the *Clostidium* mutant strains Mutant 1 (cross-hatched histograms), Mutant 2 (grey histograms) or Mutant 3 (black histograms) according to the invention.
**Figure 2** is an alternative representation of the results from Figure 1, where the acetone and PDO production results are shown on a distinct and more precise scale.

### EXAMPLES

The present invention is further defined in the following examples. It should be understood that these examples, while indicating preferred embodiments of the invention, are given by way of illustration only. From above disclosure and these examples, the person skilled in the art can make various changes of the invention to adapt it to various uses and conditions without modifying the essential means of the invention.

### Example 1: Construction of Clostridium acetobutylicum strains producing glycerol and acetate (mutant 1), glycerol, 1,3-propanediol and acetate (mutant 2)

The two mutants were constructed from the CAS2 *Δbuk-ptb* strain constructed by Wilding-Steele et al. (2021) using the CRISPR/Cas9 tool also demonstrated by Wilding-Steele et al. (2021).

First, the plasmid pGRNAΔthIA constructed by Genewiz (using a gRNA targeting *thlA* gene of sequence SEQ ID NO: 31) was transformed into the CAS2 *Δbuk-ptb* strain by electroporation. Transformants were grown in CGM with 10 g/L glucuronic acid as the sole carbon source and 15 µg/mL thiamphenicol. Then, 200 µL of the grown culture was plated onto CGM plates supplemented with 9 g/L glucuronate and 1 g/L xylose as carbon sources and 15 µg/mL thiamphenicol to allow cas9 expression and the selection of the ΔthlA mutants. The pGRNAΔthlA was then cured after two to three streaking steps on RCM plates, and the MGC *ΔCAC1502 pyrE::pxyl_cas9 Δbuk-ptb ΔthlA* strain was isolated.

The plasmid pGRNAΔldhA described by Wilding-Steele et al. (2021) was transformed into the MGC *ΔCAC1502 pyrE::pxyl_cas9 Δbuk-ptb ΔthlA* strain by electroporation. Transformants were grown in CGM with 30 g/L glucose as the sole carbon source and 15 µg/mL thiamphenicol. Then, 200 µL of the grown culture was plated onto CGM plates supplemented with 30 g/L xylose as the sole carbon source and 15 µg/mL thiamphenicol, to allow cas9 expression and the selection of the *ΔldhA* mutants. The pGRNAΔldhA was then cured after two to three streaking steps on RCM plates, and the MGC *ΔCAC1502 pyrE::pxyl_cas9 Δbuk-ptb ΔthlA ΔldhA* strain was isolated.

The plasmid pDhaBTΔpSOL was constructed from the pSPD5 plasmid described by Raynaud et al. that contains *dhaB1, dhaB2, dhaT* operon of Clostridium butyricum, repL Gram-positive replicon, colE1 Gram-negative replicon, ampicillin and erythromycin resistance markers (Raynaud et al., 2003). In pDhaBTΔpSOL, the P12009-9 strong synthetic promoter and RBS described by Yang et al. were cloned upstream of the *dhaB1, dhaB2, dhaT* operon, and a gRNA targeting solR gene (SEQ ID NO: 33) was also cloned under the control of the J23199 promoter (Yang et al., 2017). The plasmid pDhaBTΔpSOL was transformed by electroporation into the MGC *ΔCAC1502 pyrE::pxyl_cas9 Δbuk-ptb ΔthlA ΔldhA* strain. Transformants were grown in CGM with 30 g/L glucose as the sole carbon source and 25 µg/mL erythromycin. Then, 200 µL of the grown culture was plated onto CGM plates supplemented with 20 g/L glycerol and 10 g/L xylose as carbon sources and 25 µg/mL erythromycin to allow cas9 expression and the selection of the ΔpSOL1 mutants. The resulting MGC *ΔCAC1502 pyrE::pxyl_cas9 Δbuk-ptb ΔthlA ΔldhA ΔpSOL1* pDhaBTΔpSOL strain grew very well using a mixture of glycerol and glucose. It actually consumes one mole of glycerol and half a mole of glucose to produce one mole of 1,3-propanediol and one mole of acetate, it is likely that the glucose is used for acetate production while glycerol is used for 1,3-propanediol production. This strain is unable to grow on glucose as the sole carbon source because the production of acetate would accumulate NADH and the glycerol production pathway is not effective enough to compensate. Nevertheless, this substrate constraint constitutes a powerful lever for strain evolution to promote the selection of an efficient glycerol pathway. Therefore, a 30 mL grown culture of the MGC *ΔCAC1502 pyrE::pxyl_*cas9 *Δbuk-ptb ΔthlA ΔldhA ΔpSOL1* pDhaBTΔpSOL strain in CGM supplemented with 20 g/L glucose, 20 g/L glycerol and 25 µg/mL erythromycin was centrifuged at 7000 xg for 10 minutes, the pellet was washed and resuspended in CGM and then spread on CGM plates supplemented with glucose as the sole carbon source. Mutants 1 and 3 were isolated based on their ability to grow on glucose alone.

The mutant 1 has lost the pDhaBTΔpSOL plasmid and is able to grow in both the CGM rich medium and the minimal and chemically defined medium SM, producing almost one mole of glycerol and one mole of acetate per mole of glucose consumed. A low amount of biomass is produced: more than 30 g/L of glucose can be consumed with an OD600 of only 1 and the strain has a maximum growth rate of 0.15 h-1. Genomic DNA from the mutant and the parental strain MGC *ΔCAC1502 pyrE::pxyl_*cas9 *Δbuk-ptb ΔthlA ΔldhA ΔpSOL1* pDhaBTΔpSOL were extracted and sequenced using Ion Torrent Technology. This showed a large increase in sequencing reads in a region of twelve genes from *CA_C1614* to *CA_C1627* in the glycerol-producing mutant 1. Interestingly, this region includes the genes *CA_C1625* and *CA_C1626* which were identified as encoding a phosphoserine phosphatase and a glycerol dehydrogenase, respectively. Using the reverse primer pSB1016 (CTGTGGTGTCAGGGCAATGAA ; SEQ ID NO: 42) located in *CA_C1614* and the forward primer pSB1017 (GGGAAATACTTGATAATGCCATAGATGAGC ; SEQ ID NO: 43) located in *CA_C1627* we were able to amplify a 352 bp fragment suggesting that the *CA_C1614* to *CA_C1627* sequence is at least duplicated in the mutant - as nothing can be amplified in a wild-type background.

The mutant 2 was also isolated from the strain MGC *ΔCAC1502 pyrE::pxyl_cas9 Δbuk-ptb ΔthlA ΔldhA ΔpSOL1* pDhaBTΔpSOL for its ability to grow on glucose as sole carbon source. Mutant 2 retained the pDhaBTΔpSOL plasmid and is therefore able to convert glucose into glycerol, 1,3-propanediol and acetate.

### Example 2 Construction of Clostridium acetobutylicum strains producing glycerol, acetate and acetone (mutant 3)

The mutant 3 was constructed from the mutant 1. The plasmid pΔhdb::PₜₕₗthlA3_{ck}-ctfABadc_{cb} was constructed from the pGRNA vector backbone described in Wilding-Steele et al. (Wilding-Steele et al., 2021). It contains 900 bp homology regions that are flanking the *hbd* gene in the C. *acetobutylicum* chromosome, a gRNA targeting the *hbd* gene (SEQ ID NO: 32) cloned under the control of the J23199 promoter, and a synthetic operon consisting of the *thlA* promoter Pthl, *the thlA3* gene from *Clostridium kluyveri* (SEQ ID NO: 40, coding the THLA3 protein with SEQ ID NO: 41), the *ctfA, ctfB* and adc genes from *Clostridium beijerinckii* is cloned between the two homology regions (SEQ ID NO: 34 for *thlA* homology region and SEQ ID NO: 35 for *hbd* homology region). The plasmid pΔhdb::PₜₕₗthlA3_{ck}-ctfABadc_{cb} was transformed into the mutant 1 by electroporation. Transformants were grown in CGM with 30 g/L of glucose, and 200 µL of the culture was plated onto CGM plates supplemented with 30 g/L xylose as sole carbon source and 15 µg/mL thiamphenicol, allowing cas9 expression and the selection of the *Δhdb::PₜₕₗthlA3_{ck}*-*ctfABadc_{cb}* mutants. The pΔhdb::PₜₕₗthlA3_{ck}-ctfABadc_{cb} plasmid was then cured after two streaking steps on RCM plates and the mutant 3 was isolated. In this mutant the *hbd* gene is replaced by a synthetic operon allowing the conversion of acetate to acetone.

### Example 3 Construction of Clostridium acetobutylicum strains producing 1,3-propanediol, glycerol, acetate and acetone (mutant 4)

The mutant 4 was constructed from the mutant 3 by transforming it with the pSPD5 plasmid (Raynaud et al. 2003). The pSPD5 plasmid was transformed into the mutant 3 by electroporation. Transformants were grown in CGM with 30 g/L of glucose and 40µg/ml erythromycin.

When grown in CGM medium with 40µg/ml erythromycin mutant 4 was shown to produce 1,3-propanediol, glycerol, acetate and acetone.

### Example 4 Construction of Clostridium acetobutylicum strains producing 1, 3-propanediol and acetate (mutant 5) without hydrogen production

Plasmid pSPD8 was constructed from the pSPD5 plasmid (Raynaud et al. 2003) by replacing, in the synthetic operon, the *dhaT* gene by the *bdhB* and *CAC0764* genes of *C*. *acetobutylicum* encoding an NADPH dependent alcohol dehydrogenase (Yoo et al 2015) and a Fd-NADP+ reductase (Foulquier et al 2022, US10,760,100) respectively.

The pSPD8 plasmid was transformed into the mutant 1 by electroporation. Transformants were grown in CGM with 30 g/L of glucose and 40µg/ml erythromycin.

The *hydA* gene encoding the main hydrogenase of *C*. *acetobutylicum* was then deleted from this recombinant strain by transforming it with the pCAT-upp hydA plasmid and selecting for 5-FU resistant strain (US10,669,530). When grown in CGM medium with 40µg/ml erythromycin the hydrogenase minus mutant was shown to produce 1, 3 - propanediol and acetate only without production of hydrogen and only traces amount of glycerol.

### Example 5: Heterologous expression of genes CA C1625 and CA C1626 enable glycerol production in Escherichia coli

As the duplication of the *CA_C1614* to *CA_C1627* region is the only significant mutation that occurred after obtaining the glycerol-producing *C*. *acetobutylicum* mutants, we assumed that overexpression of *CA_C1625,* which encodes a hypothetical protein comparable to a glycerol phosphate phosphatase, and *CA_C1626,* which is predicted as encoding a glycerol phosphate dehydrogenase, enables glycerol production. To demonstrate this, the *CA_C1625-CA_C1626* region, together with the 35 bp sequence upstream of *CA_C1625* containing the RBS, was amplified by PCR with pSB1025 (CGTAGGATCCTAATATTTAAAAAATAGAACATGGGGGGAAATCAAATGA ; SEQ ID NO: 44) and pSB1026 (AAGTGGCGCCTTATACCAATATATTCCTTAATATTTCGTCAGTATCCACAA; SEQ ID NO: 45) and cloned into a pSOS95 backbone under the control of the Pthl promoter (Fontaine et al., 2002), using the BamHI-HF and the Sfol restriction enzymes and the T4 ligase (provided by New England biolabs), yielding to pSOS95-CAC1625-CAC1626 vector. The empty pSOS95-del vector was thereby used as a negative control (Fontaine et al., 2002).

The pSOS95-CAC1625-CAC1626 and pSOS95-del vectors were transformed by electroporation into the *E. coli* Δ*tpiA* strain constructed by Meynial-Salles et al. (Meynial Salles et al., 2007). The deletion of the triose phosphate isomerase gene *tpiA* was aimed at facilitating glycerol production, since the dihydroxyacetone phosphate produced in glycolysis cannot be converted to glyceraldehyde-3-phosphate. Transformants were selected on LB containing 100 µg/L carbenicillin. For both *E. coli* Δ*tpiA* pSOS95-CAC1625-CAC1626 strain and *E. coli* Δ*tpiA* pSOS95-del strain, six individual clones were grown in microaerobiosis on synthetic M9 medium containing 30 g/L glucose. A yield of 0.21 mol_{glycerol}/mol_{glucose} was obtained for the pSOS95-CAC1625-CAC1626 strain compared to 0.02 mol_{glycerol}/mol_{glucose} for the strain carrying the control plasmid pSOS95-del, showing that *CA_C1625* and *CA_C1626* allowed glycerol production. The other products are acetate, lactate and ethanol, which means that the chosen *E. coli* Δ*tpiA* mutant may have adapted to compensate for the loss of the triose phosphate isomerase by using the methylglyoxal or Entner Doudoroff pathways. This would allow the use of excess dihydroxyacetone phosphate or bypass the fructose-biphosphate aldolase and explain why less than one mole of glycerol is produced per mole of glucose in the pSOS95-CAC1625-CAC1626 strain and why the pSOS95-del strain is viable.

### Example 6: Culture of the Clostridium acetobutylicum mutants

An experiment carried out in which the mutant 1 (*C*. *acetobutylicum* MGC *ΔCAC1502 pyrE::pxyl_cas9 Δbuk-ptb ΔthlA ΔldhA ΔpSOL1 CAC1625-CAC1626* duplicated) was cultured in the synthetic medium SM (Soni et al., 1987), in a 300 mL fermenter pH-regulated to 5.5 by the addition of ammonia. This showed a production of 28.3 g/L of glycerol and 16.5 g/L of acetate from 62 g/L of glucose with a co-production of acetoin (0.8 g/L) and traces of lactic acid, within 78 h. The maximum glycerol productivity was 0.53 g.L-1.h-1 (12.7 g.L-1.day-1 ) during the exponential growth phase, which is equivalent to the values obtained by some industrially relevant yeast fermentations and higher than the compared anaerobic processes (Semkiv et al., 2017, 2020). Furthermore, the yield of 0.46 g_{glycerol}/g_{glucose} is close to the theoretical one as almost one mole of glycerol is produced from one mole of glucose. Metabolism is limited by the production of acetate, which is needed to produce NADH and ATP. As glucose is mainly imported via the phosphotransferase system (PTS), one mole of PEP must be produced for each mole of glucose consumed, explaining the 1 mol_{acetate}/mol_{glucose} ratio.

A similar experiment was performed using the mutant 3 (*C*. *acetobutylicum* MGC *ΔCAC1502 pyrE::pxyl_cas9 Δbuk-ptb ΔthlA ΔldhA ΔpSOL1 CAC1625-CAC1626* duplicated *Δhdb::PₜₕₗthlA3_{ck}-ctfABad_{cb}*) cultured in the synthetic medium SM with 60 g/L of glucose in a 300 mL fermenter pH-regulated to 5.0 by the addition of ammonia. This pH has been shown to optimise the flux through the CoA transferase, thereby enhancing acetone production (Dusséaux et al., 2013). This resulted in 18.6 g/L glycerol, 8.1 g/L acetate, 1.2 g/L acetone and 0.7 g/L acetoin being produced from 38 g/L of glucose in a hundred hours, with a maximum OD600 below 1.1. This showed that the implemented thlA3_{ck}-ctfABadc_{cb} acetone pathway allows partial conversion of acetate to acetone. We assumed that growth stopped after 72 h due to toxic acetate accumulation, so the acetone pathway needs to be improved by selecting other genes or increasing their expression levels to allow more efficient acetate uptake.

Another experiment was performed with the mutant 2. Four cultures were performed on rich medium (CGM) supplemented with 50 g/L of glucose in serum bottles with the pH adjusted to around 6 by the addition of ammonia. It was found that after 72 to 125 hours of culture, 29 g/L glucose yielded an average production of 6.5 g/L glycerol, 4 g/L 1,3-propanediol and 10 g/L acetate. This means that one third to one half of the glycerol produced from glucose was directly converted to 1,3-propanediol. Interestingly, the strain produced 1,3-propanediol even when the antibiotic pressure of the plasmid is not maintained. In this culture, the NADH produced by acetate and acetoin formation completely balances the demand for glycerol and 1,3-propanediol synthesis. This means that all the reduced ferredoxin is re-oxidised by the hydrogenase, resulting in the formation of dihydrogen.

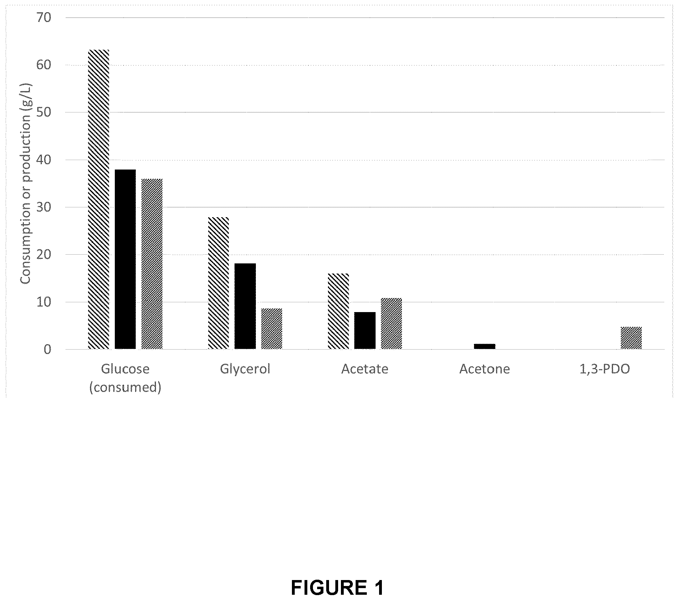

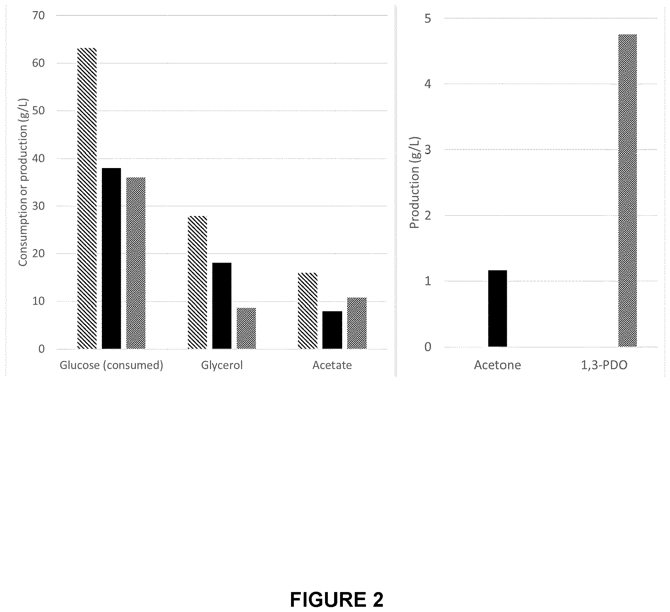

These results are presented in Figures 1 and 2.

### NON-PATENT REFERENCES

- YOO M, BESTEL-CORRE G, CROUX C, RIVIERE A, MEYNIAL-SALLES I, and SOUCAILLE P*. (2015) A Quantitative System-Scale Characterization of the Metabolism of Clostridium acetobutylicum. MBio. 6: e01808-15
- FOULQUIER C, RIVIERE A, HEULOT M, DOS REIS S, PERDU C, GIRBAL L, PINAULT M, DUSSEAUX S, SOUCAILLE P*, and MEYNIAL-SALLES I. (2022) Molecular characterization of the missing electron pathways for butanol synthesis in Clostridium acetobutylicum. Nature Com. 13, 4691
- WILDING-STEELE, T., RAMETTE, Q., JACOTTIN, P., & SOUCAILLE, P. (2021). Improved CRISPR/Cas9 Tools for the Rapid Metabolic Engineering of Clostridium acetobutylicum. International Journal of Molecular Sciences, 22, 3704. https://doi.org/10.3390/ijms22073704
- RAYNAUD, C., SARÇABAL, P., MEYNIAL-SALLES, I., CROUX, C., & SOUCAILLE, P. (2003). Molecular characterization of the 1,3-propanediol (1,3-PD) operon of Clostridium butyricum. Proceedings of the National Academy of Sciences of the United States of America, 100(9), 5010-5015. https://doi.org/10.1073/pnas.0734105100
- YANG, G., JIA, D., JIN, L., JIANG, Y., WANG, Y., JIANG, W., & GU, Y. (2017). Rapid Generation of Universal Synthetic Promoters for Controlled Gene Expression in Both Gas-Fermenting and Saccharolytic Clostridium Species. ACS Synthetic Biology, 6, 1672-1678. https://doi.org/10.1021/acssynbio.7b00155
- FONTAINE, L., MEYNIAL-SALLES, I., GIRBAL, L., YANG, X., CROUX, C., & SOUCAILLE, P. (2002). Molecular characterization and transcriptional analysis of adhE2, the gene encoding the NADH-dependent aldehyde/alcohol dehydrogenase responsible for butanol production in alcohologenic cultures of Clostridium acetobutylicum ATCC 824. Journal of Bacteriology, 184(3), 821-830. https://doi.org/10.1128/jb.184.3.821-830.2002
- MEYNIAL SALLES, I., FORCHHAMMER, N., CROUX, C., GIRBAL, L., & SOUCAILLE, P. (2007). Evolution of a Saccharomyces cerevisiae metabolic pathway in Escherichia coli. Metabolic Engineering, 9(2), 152-159. https://doi.org/10.1016/j.ymben.2006.09.002
- SEMKIV, M. V., DMYTRUK, K. V., ABBAS, C. A., & SIBIRNY, A. A. (2017). Metabolic engineering for high glycerol production by the anaerobic cultures of Saccharomyces cerevisiae. Applied Microbiology and Biotechnology, 101(11), 4403 4416. https://doi.org/10.1007/s00253-017-8202-z
- SEMKIV, M. V., RUCHALA, J., DMYTRUK, K. V., & SIBIRNY, A. A. (2020). 100 Years Later, What Is New in Glycerol Bioproduction? Trends in Biotechnology, 38(8), 907 916. https://doi.org/10.1016/j.tibtech.2020.02.001
- SONI, B. K., SOUCAILLE, P., & GOMA, G. (1987). Continuous acetone-butanol fermentation: Influence of vitamins on the metabolic activity of Clostridium acetobutylicum. Applied Microbiology and Biotechnology, 27(1), 1 5. https://doi.org/10.1007/BF00257244
- DUSSEAUX, S., CROUX, C., SOUCAILLE, P., and MEYNIAL-SALLES, I. (2013) Metabolic engineering of Clostridium acetobutylicum ATCC 824 for the high-yield production of a biofuel composed of an isopropanol/butanol/ethanol mixture. Metab Eng.18:1-8.
- BANTSCHEFF, M., SCHIRLE M., SWEETMAN, G., RICK, J., and KUSTERET, B., (2007), Quantitative mass spectrometry in proteomics: a critical review. Analytical and Bioanalytical Chemistry, vol. 389(4): 1017-1031.
- BURNETTE, (1981). "Western Blotting": Electrophoretic Transfer of Proteins from Sodium Dodecyl Sulfate-Polyacrylamide Gels to Unmodified Nitrocellulose and Radiographic Detection with Antibody and Radioiodinated Protein A Analytical Biochemistry, 112(2): 195-203.
- DATSENKO, KA., and WANNER, BL., (2000), Proc Natl Acad Sci USA., 97: 6640-6645.
- ENGVALL, E., and PERLMAN, P., (1981), Immunochemistry, 8: 871-874.

## Claims

1. A method for the production of glycerol and acetate comprising culturing, in an appropriate culture medium, a *Clostridium* mutant strain comprising an overexpression of at least one of glycerol phosphate phosphatase and glycerol phosphate dehydrogenase activity.

2. The method according to claim 1, wherein said glycerol phosphate phosphatase is encoded by the gene *CA_C1625* at the locus 1764289 in the *C*. *acetobutylicum* genome ATCC824, Genbank AE001437 with SEQ ID NO: 1.

3. The method according to claim 1 or 2, wherein said glycerol phosphate dehydrogenase is encoded by the gene *CA_C1626* at the locus 1764945 in the *C*. *acetobutylicum* genome ATCC824, Genbank AE001437 with SEQ ID NO: 3.

4. The method according to any one of claims 1 to 3, wherein at least one gene encoding an enzyme involved in production of metabolites from glycerol is deleted in the *Clostridium* mutant strain, the gene being chosen among:
- *ptb* gene encoding phospho-transbutyrylase to avoid butyrate formation,
- *buk* gene encoding butyrate kinase to avoid butyrate formation,
- *Idh* gene encoding lactate dehydrogenase to avoid lactate formation,
- *adhE1* and *adhE2* genes encoding aldehyde-alcohol dehydrogenase to avoid ethanol and butanol formation.

5. The method according to claim 4, wherein the *adhE1* and *adhE2* genes are deleted by curing the *Clostridium* mutant strain for the pSOL1 megaplasmid.

6. The method according to any one of claims 1 to 5 wherein the *Clostridium* mutant strain is modified to convert acetate to acetone by overexpressing at least one gene selected among:
- *ctfA* gene from a solventogenic *Clostridium* and encoding CoA-transferase subunit A,
- *ctfB* gene from a solventogenic *Clostridium* and encoding CoA-transferase subunit B,
- adc gene from a solventogenic *Clostridium* and encoding coenzyme A-acylating aldehyde dehydrogenase.

7. The method according to claim 6, wherein the *hbd* gene encoding 3-hydroxybutyryl-CoA dehydrogenase is deleted in the *Clostridium* mutant strain.

8. The method according to any one of claims 1 to 7, wherein the *Clostridium* mutant strain is modified to produce 1,3-propanediol from glycerol by overexpressing the genes:
- *dhaB1* gene from *Clostridium butyricum* and encoding B12-independent glycerol dehydratase,
- *dhaB2* gene from *Clostridium butyricum* and encoding glycerol dehydratase activator, and
- either *dhaT* gene from *Clostridium butyricum* and encoding 1,3-propanediol dehydrogenase or both *bdhb* gene from *Clostridium acetobutylicum* encoding an NADP+ dependent alcohol dehydrogenase and *CA_C0764* gene from *Clostridium acetobutylicum* encoding a Fd-NADP+ reductase.

9. The method according to claim 8, wherein the *hydA* gene encoding hydrogenase I is deleted in the *Clostridium* mutant strain.

10. The method according to any one of claims 1 to 9, wherein said *Clostridium* strain is selected from the group consisting of *Clostridium acetobutylicum, Clostridium butyricum, Clostridium beijerinckii, Clostridium pasteurianum and Clostridium thermocellum.*

11. The method according to any one of claims 1 to 10, wherein the source of carbon in the culture medium is glucose, fructose, xylose and/or sucrose, preferably glucose.

12. The method according to any one of claims 6 to 11, further comprising recovering the 1,3-propanediol and/or acetone produced from the culture medium.

13. A mutant strain of *Clostridium* comprising an overexpression of at least one of glycerol phosphate phosphatase encoded by the gene *CA_C1625* at the locus 1764289 in the *C*. *acetobutylicum* genome ATCC824, Genbank AE001437 with SEQ ID NO: 1 and glycerol phosphate dehydrogenase encoded by the gene *CA_C1626* at the locus 1764945 in the *C*. *acetobutylicum* genome ATCC824, Genbank AE001437 with SEQ ID NO: 3.

14. The mutant strain of *Clostridium* as defined in anyone of claims 4 to 10.

15. The mutant strain according to any one of claims 13 to 14, wherein said microorganism is selected from species of the genus *Clostridium,* preferably selected from the group consisting of *Clostridium acetobutylicum, Clostridium butyricum, Clostridium beijerinckii, Clostridium pasteurianum, and Clostridium thermocellum.*
